# EUROPEAN PATENT SPECIFICATION

(11) **EP 0 601 585 B1**
(45) Date of publication and mention of the grant of the patent: **19.05.2010**
(21) Application number: 93119894.9
(22) Date of filing: 09.12.1993
(51) Int. Cl.: C12N 15/11, C12N 1/20, C12N 15/85, A61K 31/70, A01K 67/027, C12N 5/10

(54) **Nucleic acid construct for inhibiting or regulating the functions of an immuno response gene, method utilizing the same and immunologically compatible system containing the same**
Nucleinsäurekonstrukt zur Hemmung oder Regulierung der Funktionen eines Immunantwortgens, dessen Verwendung in einem Verfahren und immunologisch kompatibles System, das dieses Konstrukt enthält
Acide nucléique pour l'inhibition ou la régulation des fonctions d'un gène de la réponse immunitaire, méthode employant le-dit acide nucléique et système immuno-compatible contenant le-dit acide nucléique

(30) Priority: 09.12.1992 US 988256
(43) Date of publication of application: 15.06.1994
(73) Proprietor: ENZO THERAPEUTICS, INC., Farmingdale, New York 11735-4716 (US)
(72) Inventor: Rabbani, Elazar, New York, New York 10003 (US)
(74) Representative: Vossius & Partner

(56) References cited:
- EP-A- 0 231 495
- EP-A- 0 439 208
- WO-A-89/05855
- WO-A-91/15503
- WO-A-91/18088
- WO-A-93/14769
- WO-A-93/21202
- J. EXPER. MED., vol.169, no.1, 1 January 1989 pages 351 - 356 V. LOTTEAU ET AL. 'Modulation of HLA II antigen expression by transfection of sense and antisense DR-alpha cDNA'
- ANTICANCER DRUG DESIGN, vol.7, no.4, August 1992, ENGLAND pages 341 - 350 M. KANBE ET AL. 'Influence on metastasis of the reduction of MHC class I gene with an antisense oligonucleotide'
- NATURE, vol.342, 23 November 1989, LONDON pages 435 - 438 M. ZIJLSTRA ET AL. 'Germ-line transmission of a disrupted beta2-microglobulin gene produced by homologous recombination in embryonic stem cells'
- NATEURE, vol.347, 25 October 1990, LONDON pages 770 - 772 H. BROWNE ET AL. 'A complex between the MHC class I homologue encoded by HCMV and Beta2-microglobulin'

## Description

### FIELD OF THE INVENTION

This invention relates to the field of immunology and molecular biology, including antisense technology. More particularly, it is concerned with the use for rendering a biological system - be it an non-human organism, organ, tissue, or cells - immunologically compatible - using antisense-based nucleic acid constructs. Immunological compatibility is achieved by introducing a nucleic acid construct into the cells of a system. When present in such cells, the nucleic acid construct inhibits or regulates the function of an immune response (Ir) gene, thereby rendering such a system immunologically compatible. The present invention is concerned with a nucleic acid construct as defined infra for rendering a system immunologically compatible and is also concerned with an immunologically compatible system containing a nucleic acid construct as described herein. This may be applicable to the related fields of transplantation, transfusion and therapies aimed at the alleviation or elimination of autoimmune disease symptoms.

### BACKGROUND OF THE INVENTION

The term immune response is used to describe the adaptive response of an organism or part of an organism, e.g., organ, to non-self determinants, including, for example, antigens or hapten-antigen conjugates even though the antigen may have no relationship to immunity or disease. For background material on immunology, see Immunology: A Short Course (2nd edition), E. Benjamini and S. Leskowitz, Wiley-Liss, Inc., New York, 1991; Basic Human Immunology, D. Stites and A. Terr, Appleton & Lange, Norwalk, CT, 1991; and Basic and Clinical Immunology, D. Stites and A. Terr, Appleton & Lange, Norwalk, CT, 1991.

Although the ability to evoke an immune response is a crucial aspect of animal survival, it is nevertheless highly desirable in many therapeutic applications to impart a state of immune acceptability or immune compatibility in a patient or subject. Treatment of autoimmune disease represents one such application setting where the amelioration of symptoms can be achieved by reducing the patient's ability to elicit an immune response. Autoimmune diseases represent a group of disorders in which the subject's own immune system mistakenly identifies its own tissue as foreign and consequently mounts an attack against that tissue. In the United States alone, autoimmune diseases affect nearly four million people. Notable among human autoimmune diseases are Graves' disease, multiple sclerosis, rheumatoid arthritis, myasthenia gravis, lupus erythematous, celiac disease and Crohn's disease.

In the area of organ transplantation, the recipient patient's survival is closely tied to adequate immune suppression, which if not maintained for an adequate period, can lead to organ rejection and the inevitable demise of the patient. For background on the subject of organ transplant pathology, see The Pathology of Organ Transplantation, George E. Sale, M.D., editor, Butterworths, Boston, 1990. A number of immunosuppressive therapies have been investigated for their application to organ transplants. Among these are physical immunosuppression, e.g., radiation (x-rays, gamma rays); surgery (thymectomy); chemical methods (corticosteroids or glucosteroids, purine analogs, e.g., azathioprine, and pyrimidine analogs; folic acid antagonists (for example, aminopterin, amethopterin and methotrexate); alkylating agents (notably cyclosporin, rapamycin, FK506, and related derivatives); enzyme inhibitors, as well as a number of miscellaneous agents (cytotoxic compounds, e.g., puromycin, actinomycin D, mitomycin C, ribonuclease, asparaginase, plant alkaloids, e.g., colchicine, vincristine and vinblastine; biologic methods (e.g., immunologic tolerance); antigen competition; anti-T cell monoclonal antibodies, antilymphocyte serum or globulin (ALS or ALG), or antithymocyte serum or globulin (ATS or ATG). A good discussion of immunosuppresive therapies is provided by Barrett (Textbook of Immunology: An Introduction to Immunochemistry and Immunobiology, 4th ed., C.V. Mosby Company (St. Louis, MO), chapter 11, "Transplantation Immunology," page 236, 1983).

Other procedures including blood transfusions and bone marrow transplants represent a therapeutic area where immunological compatibility is also highly desirable if severe reaction is to be avoided or minimized. Transfusion reactions most commonly occur because of the reaction between the recipient's antibodies and the transfused cells from the donor. To avoid transfusion reactions, the donor and recipient blood groups are typically cross-matched, primarily for ABO and Rh compatibility. Bone marrow transplants have been used in the treatment of a number of diseases, including severe combined immunodeficiency disease (SCID), aplastic anemia, leukemias, lymphomas, and selected solid tumors. It has long been recognized that matching the donor and the recipient at the MHC loci reduced the incidence of graft-versus-host (GVH) disease. In the past, most donors for bone marrow transplantation have been either identical twins (syngeneic) or genotypically HLA-identical individuals (allogenic). With only 40% of patients on average being expected to have an HLA-identical donor, investigations naturally turned to the use of bone marrow from partially matched family members or phenotypically matched unrelated donors. Such investigations have involved the use of high-dose chemoradiotherapy and even more lately, the use of monoclonal antibodies to leukemic and other malignant cells.

One avenue of investigation aimed at modifying a subject's immunogenicity (ability to evoke an immune response) has been to block or mask histocompatibility antigens in the donor. Faustman et al. ("Prevention of Xenograft Rejection by Masking Donor HLA Class I Antigens," Science, 252:1700-1702, 1991) disclose that the rejection of xenogeneic human pancreatic islets and liver was circumvented by masking before transplantation the donor antigens with F(ab')2 antibody fragments to HLA (Human Leucocyte Antigens) class I or tissue-specific epitopes. According to the authors, interruption of recipient T cell recognition by concealment of foreign HLA class I determinants allowed xenograft survival to continue for at least 200 days. As a general approach, however, antigen masking with antibodies suffers from a number of drawbacks. For one, the treatment is temporary at best. For another, the antibodies may elicit an immune response of their own.

Other studies have been undertaken which relate to the role of the Major Histocompatibility Complex (MHC) in transplant rejection. Hoglund et al. ("Recognition of β2-microglobulin-negative (β2m-) T-cell blasts by natural killer cells from normal but not from β2m- miceNonresponsive-ness controlled by βm- bone marrow in chimeric mice," Proc. Natl. Acad. Sci. (USA), 88: 10332-10336 (1991)) draw three conclusions from their study. First, homozygous loss of the β₂m gene coding for the light chain of the class I molecule, is sufficient to render some normal cells susceptible to recognition by NK cells. Second, NK cells of β₂m⁻/⁻ mice have lost ability to kill normal β₂m-deficient target although standard target recognition may still persist. Third, β₂m/MHC class I deficiency in the hematopoietic system (β₂m⁻/⁻ to B6 chimeras) is sufficient to calibrate the NK cells to tolerate normal β₂m⁻/⁻ mice (in contrast to the selection of CD8 + T cells, also deficient in β₂m⁻/⁻ mice) which can occur under conditions where class I expression is present only in nonhemato-poietic cells. Muller et al. ("LCMV-Specific, Class II-Restricted Cytotoxic T Cells in β2-Microglobulin-Deficient Mice," Science, 255:1576-1578 (1992)) report that β₂-Microglobulin-deficient mice (β₂M⁻/⁻) do not express functional MHC class I proteins and do not produce significant numbers of CD8⁺ T cells. When β₂M⁻/⁻ mice were infected with LCMV (lymphocytic choriomeningitis virus), many of the infected mice died from the disease and produced a specific response to LCMV mediated by CD4⁺ CTLs that were class II-restricted, suggesting that in such mice, CD4⁺ CTLs may compensate for the lack of CD8⁺ CTLs. Tarleton et al. ("Susceptibility of β2-microglobulin deficient mice to Trypanosoma cruzi infection," Nature, 356:338-340 (1992)) obtained results showing that β₂m⁻ mice suffer high cytoplasmic protozoan parasite *Trypanosoma cruzi,* and that despite such an increased susceptibility, the β₂m⁻ mice are more responsive than their β₂⁻m⁺ littermates in terms of lymphokine production (higher levels of both interleukin-2 and interferon-gamma being produced in response to mitogen stimulation).

A number of findings have been reported by researchers from the Whitehead Institute for Biomedical Research. Bix et al. ("Rejection of class I MHC-deficient haemopoietic cells by irradiated MHC-matched mice," Nature, 349:329-331 (1991)) report that NK1.1 + cells in normal mice reject haemopoietic transplants from mice that are deficient for normal cell-surface MHC-I expression because of a targeted mutation in the β₂-microglobulin gene, demonstrating that deficient expression of MHC-I molecules render marrow cells susceptible to rejection. Liao et al. ("MHC Class I DeficiencySusceptibility to Natural Killer (NK) Cells and Impaired NK Activity," Science, 253:199-202 (1991) disclose that normal T cell blasts from MHC class I-deficient mutant mice were found to serve as target cells for NK cells in vitro, suggesting that MHC class I molecules are directly involved in NK cell recognition. Pereira et al. ("Blockade of transgenic (gamma-delta) T cell development in β2-microglobulin deficient mice," EMBO, 11:25-31 (1992)) found that no mature transgenic (Tg) (gamma-delta) T cells were detected in the thymus or the spleen of β₂m⁻ (gamma-delta) Tg mice after Tg mice for the (gamma-delta) T cell receptor (TCR) were crossed with mice genetically deficient in β₂-microglobulin. Zijlstra et al. ("Skin Graft Rejection by β2-microglobulin-deficient Mice," J. Exp. Med., 175:885-893 (1992)) concluded from their results on β₂-microglobulin-deficient mice that MHC-I-directed CD8⁺ CTLs are not essential in the rejection of allografts with whole MHC or multiple minor H differences, but that the absence of MHC-I-guided immunity profoundly reduces the ability of mutant mice to reject H-Y disparate grafts. In an earlier published work, Zijlstra et al. ("β2-Microglobulin deficient mice lack CD4-8+ cytolytic T cells," Nature, 344:742-746 (1990)) disclose that mice homozygous for a β₂-microgloubulin gene disruption do not express any β₂-m protein, and further, that they express little if any functional MHC complex class I antigens on the cell surface. In addition, such mice show a normal distribution of gamma-delta CD4⁺8⁺ and CD4⁺8⁻ T cells but have no mature CD4⁻8⁺ T cells and are defective in cD4⁻8⁺ T cell-mediated cytotoxicity. Previously, Zijlstra et al. ("Germ-line transmission of a disrupted β2-microglobulin gene produced by homologous recombination in embryonic stem cells," Nature, 342:435-438 (1989)) reported that the introduction of a targeting vector into embryonic stem cells resulted in β₂-m gene disruption with high frequency, and that chimeric mice derived from blastocytes injected with mutant embryonic stem cell clones transmit the mutant allele to their offspring.

Other investigations have been reported. A group led by David H. Sachs of the Transplantation Biology Research Center, Massachusetts General Hospital, has examined specific transplantation tolerance in several species, including pig, mouse and rat. In a 1991 review article, Smith et al., including D.H. Sachs ("New Approaches to Transplantation Tolerance," Transplantation Proceedings, 23:2157-2161 (1991)) disclose a combination of protocols in which bone marrow transplant serves to achieve tolerance to class II antigens disparities while a short course of cyclosporin A (CyA) would induce tolerance to class I antigens and minor antigen disparities. In a later published letter to the editor, Sachs discusses the induction of mixed chimerism as a means for producing specific transplantation tolerance in mice while averting graft-versus-host disease and retaining full immunocompetence. According to the author, mixed chimeric states have been achieved by various procedures including the followingtotal lymphoid irradiation, reconstitution of lethally irradiated mice with mixtures of T-cell-dependent bone marrow from the host and T-cell-depleted bone marrow from the donor, nonmyeloablative preparative regimen involving the use of monoclonal antibodies against T-cell subgroups and sublethal irradiation to prepare the recipient for bone marrow transplantation. Shafer et al. ("Expression of a swine class II gene in murine bone marrow hematopoietic cells by retroviral-mediated gene transfer," Proc. Natl. Acad. Sci. USA, 88:9760-9764 (1991)) conclude from their study that a significant proportion of very primitive myelopoietic murine precursor cells can be transduced with a DRB recombinant vector and that the vector sequences are expressed in the differentiated progeny of the cells. Aksentijevich et al. ("Natural Antibodies Can Inhibit Bone Marrow Engraftment in the Rat--> Mouse Species Combination," J. Immunol., 147:4140-4146 (1991)) disclose that normal murine sera containing high levels of cytotoxic natural antibody against rat bone marrow cells (BMC) are capable of inhibiting engraftment of rat BMC in mice. Rosengard et al. ("The Failure of Skin Grafting to Break Tolerance to Class I-Disparate Renal Allografts in Miniature Swine Despite Inducing Marked Antidonor Cellular Immunity," Transplantation, 52:1044-1052 (1991)) disclose that tolerance of renal allografts in miniature swine cannot be broken with multiple skin grafts despite bearing donor class I plus third-party class II antigens, despite the ability of the first of such grafts to induce alloaggression. Skin grafting effectively augmented donor-specific cell-mediated immunity, however, thus ruling out clonal deletion as a mechanism.

The problems of contamination and infection in the field of human blood transfusions have classically been a matter of concern, and with the advent of HIV epidemiology, they have become particularly troublesome. Of more recent concern is the possible transmission of HIV infections in organ and tissue transplantation. In a March 22, 1992 broadcast on the Lifetime cable television program, "Internal Medicine Update," the risk of Human Immunodeficiency Virus (HIV-I) transmission through transplantation was discussed among several experts in related fields. A transcript and videocassette of that broadcast was made available by Burrelle's Information Services, P. O. Box 7, Livingston, NJ 07039. During this broadcast, the transmission incidence was described as exceedingly low, based, for example, on only three reports of HIV transmission following more than 60,000 organs and one million tissue transplants. The incidence may, in fact, be higher than reported because some of the transplant recipients may have been exposed to HIV-infected organs and tissues during the so-called "window period" when infection is possible despite the seeming absence of antibodies. Despite such a low reported incidence, the possibility of HIV transmission in organ and tissue transplantation remains a matter of acute concern, given the fact that the window period for HIV infection generally lasts at least from six to eight weeks. Another factor that adds to this concern is the fact that an explanation may be available to account for transmission in some tissue recipients, but not in others. In addition, the need for adequate screening of donors and the possibility of attendant errors in the compilation and monitoring of donors in an area where organ and tissue candidates are continually in short supply relative to the number of potential recipients, raises further concern among medical transplant practitioners and their patients.

WO 93/21202, intermediate art under Article 54(3)(4) EPC 1973, relates to synthetic (partially) phosphorothioated oligonucleotides capable of selectively modulating hemopoietic bone marrow cells development. Synthetic antisense oligonucleotides are provided directed against the human ACHE, 2HS, BCHE and CHED (cdc2 homolog) The application also relates to pharmaceutical compositions thereof.

WO 93/14769, cited under Article 54(3)(4) EPC 1973, refers to transplantation antigen-depleted cells made by exposing a target cell to an oligonucleotide capable of binding to a transplantation antigen (MHC class I or II) nucleotide sequence. The oligonucleotide is capable of binding to the nucleotide sequence according to Watson-Crick or triplex binding rules. Specific oligonucleotides useful in practicing this method for making donor cells are also provided.

Lotteau et al., 1989 J. Exp. Med. 169 (1): 351-356 report on the modulation of HLA class II antigen expression by transfection of sense / antisense DRα cDNA and use a monovalent nucleic acid construct confined to a single portion of the target gene sequence.

Kanbe et al., 1992 Anticancer Drug Design 7: 341-350 describe the influence on metastasis by the reduction of MHC Class I gene expression based on an antisense strategy. However, the authors use monovalent unmodified oligonucleotides, unlike the present invention.

Further, specific soluble suppressor factor (SSF) sequences which are capable of specifically inhibiting T-cell immune responses are described in WO 91/15503.

Many of the afore-described methodologies aimed at reducing a subject's immunogenicity rely on drastic procedures which are typically reserved for high risk cases. Furthermore, even if one took for granted that there was a genetic basis to the immune response mechanism, it was not at all clear from the prior art - given the diversity of possible genetic factors - that a manageable number of genes controlling the immune response could be usefully manipulated to achieve immunological compatibility in a biological system. Moreover, given the various approaches to genetic manipulation, both transient and permanent, including insertion and deletion processes, and further given the complexity of gene structures and the number and diversity of structures in an organism's genome, it was not apparent given all the ammunition for genetic manipulation, that one could still achieve either sufficient regulation in a given gene and sufficient regulation in the complex animal system in order to control the manifestation of immune response. Even given the enormous diversity in the genomic basis of the immune response, it was not clearly recognized in the prior art there was sufficient homologies in the regions of those determinants, that one could come forward with a practical resolution of genetic regulation of the immune response. The present invention is based in large part on the discovery and recognition that immune response genes can be effectively regulated or controlled, notwithstanding all of the aforementioned variables which contribute to the enormous diversity and complexity of the immune response mechanism.

### SUMMARY OF THE INVENTION

The present invention provides a nucleic acid construct comprising a plurality of oligonucleotides complementary to different portions of a ribonucleotide sequence transcribed from an immune response gene in a system which construct when present in a cell, inhibits or regulates the function of said gene, wherein said immune response gene codes for: (a) Class I or Class III antigens of the major histocompatibility complex, or (b) cell adhesion molecules, and wherein said complementary oligonucleotides comprise at least one modified member selected from the group consisting of an association constant increasing modifier and an association increasing modifier, or a combination of any of the foregoing.

The present invention also provides for the use of said nucleic acid construct for rendering a system, e.g., a non-human organism, organ, tissue, or cells, immunologically compatible. The use comprises that into the cells of such a system said nucleic acid construct is to be introduced which inhibits or regulates the function of an immune response (Ir) gene or genes responsible for the immunological determinants and/or for controlling an immune response. The nucleic acid construct can usefully take a number of forms conducive to genetic intervention, including, for example, genetic antisense or antisense oligonucleotides ("antisense oligos"), as well as nucleic acid triplex formation, point mutations, nucleic acid modifiers and co-suppression (sense) technology.

Further described are intercalators, e.g. ethidium bromide.

Further provided by the present invention is the use for rendering a system immunologically compatible by introducing into cells, e.g., non-human germ cells, fertilized cell, non-human embryonic cells and somatic cells, a nucleic acid construct as defined above which, when present in a cell, inhibits or regulates the function of an immune response (Ir) gene or genes in the system, and thereafter, allowing the cells to develop or maturate further into an immunologically compatible system.

The present invention provides a nucleic acid construct and an immunologically compatible system containing a nucleic acid construct, which construct as defined above, when present in a cell, inhibits or regulates the function of an immune response (Ir) gene or genes that determine immunological compatibility and/or control the immune response, including the immune response to specific antigens.

### DETAILED DESCRIPTION OF THE INVENTION

The present invention is predicated in large part on three - principles relating to the immunological determinants of a biological system which result in an immune response. The first principle concerns the nature of the diversity of genes involved in the immune response mechanism. Given that the immunological determinants involved in the immune response are represented by an enormously diverse universe of genes, it is nevertheless recognized in the present invention that there are sufficient homologies and common areas in the immune response genes which might serve as points of genetic control. In recognition of the homologies and common areas in the immune response genes, the second principle states that genetic intervention can be usefully applied to regulate or inhibit the immune response genes in a way that is manageable in a practical sense. A third principle which forms the basis of the present invention is that the regulation or inhibition, including elimination of the genetic determinants through genetic intervention does not substantially impair the function of a biological system, again in a practical sense, although the effects of genetic intervention may represent a limitation to the system, such as an increased but manageable susceptibility to infection. In all instances, however, the regulation and inhibition of the immune response genes through genetic intervention must not destroy or substantially impair the biological function of the system.

Having a genetic basis, the immunological determinants, or stated otherwise, the genes which control the immunological determinants, can be manipulated through genetic intervention, for example, through the application of antisense technology, to render a biological system immunologically compatible. For a concise discussion of genetic therapeutic approaches, see Gene Therapy: Application of Molecular Biology, James W. Larrick and Kathy L. Burck, Elsevier Science Publishing Co., Inc., New York, 1991. For background on the application of recombinant DNA technology on human disorders, including clinical models, see The New Genetics and Clinical Practice, D. J. Weatherall, Oxford University Press, 3rd edition, New York, 1991. In effect, the system can be changed in accordance with the present invention from one which elicits a given immunological response to one which does not manifest such a response. The genetic intervention can be usefully applied in accordance with the present invention to regulate, eliminate, inhibit, suppress or otherwise modify the function of any gene or genes responsible for controlling the immunological determinants of a system. Genetic intervention can take a number of forms, including antisense technology, nucleic acid triplex strand formation. Further described are site-specific insertions, nucleic acid modifiers or intercalators, e.g., ethidium bromide, and sense technology, i.e., co-suppression.

The terms regulate and regulation are intended to encompass genetic intervention that eliminates a genetic determinant, but also that which will introduce a new genetic determinant into the system, in combination with the elimination of some other genetic determinant. The genetic determinant can be introduced by expression (inducible or non-inducible, i.e., constitutive) and cloning techniques well know to those skilled in the art. Such techniques for introducing the genetic determinant include those disclosed by Inouye et al., *supra,* such as vectors, plasmids, delivery systems, and viruses, particularly retroviruses, physical techniques, such as electroporation and direct chromosome transfer by cell fusion. In transplantation, human HLA class I genes may be introduced into a donor devoid of class I and class II genes, in effect, a knockout class I and class II donor. In such an instance, a nucleic acid construct can be usefully administered to introduce the class I genes. Thus, the regulation is directed to both the elimination and the introduction of genetic determinants involved in the immune response.

Nucleic acids that complement the sense (coding) nucleic acid strand have been termed antisense. A good discussion of antisense principles and applications is presented in the publication, Antisense RNA and DNA, James A. H. Murray, editor, Wiley-Liss, New York, 1992. Among its various topics are antisense RNA, antisense DNA, case studies on the control of RNA viruses, *c-myc* expression in HL-60 cells and ribozymes. Another publication dealing with the subject of antisense is Discoveries in Antisense Nucleic Acids, Advances in Applied Biotechnology Series, volume 2, Christine L. Brakel, Ph.D, editor, Portfolio Publishing Company (The Woodlands, TX), 1989. Antisense nucleic acids can bind specifically to their complementary sense strands, thereby interfering with the gene function coded by the sense strand. Such gene function includes, for example, the formation of the protein encoded by the sense strand. The use of antisense nucleic acids to control gene function are broadly classified into two categories, genetic antisense and antisense oligonucleotides ("antisense oligos"), both of which may be applicable as described herein.

The regulation of gene expression based upon the utilization of nucleic acid complementary to messenger RNA (otherwise known as genetic antisense) was first disclosed by Inouye et al., U.S. Patent Application Serial No. 07/436,598, filed on November 15, 1989, now allowed, and U.S. Patent Appl. Ser. No. 07/542,209, filed on June 21, 1990 as a continuation of the former, also now allowed. The former application (07/436,598) was a continuation-in-part of U.S. Patent Appl. Ser. No. 07/300,741, filed on January 23, 1989, which in turn, was a continuation-in-part of U.S. Patent Appl. Ser. No. 07/228,852, filed on August 3, 1988, and a continuation of U.S. Patent Appl. Ser. No. 07/585,282, filed on March 1, 1984, abandoned. The latter abandoned application was a continuation-in-part of U.S. Patent Appl. Ser. No. 07/543,528, filed on October 20, 1983, abandoned, and a continuation of U.S. Patent Appl. Ser. No. 07/543,582, filed on October 20, 1983, also abandoned. Much if not all of the contents of the two last-mentioned Inouye et al. disclosures (Serial Nos. 07/543,528 and 07/585,282) was published as European Patent Appl. Publ. No. 0 140 308 (A2), published on May 8, 1985.

Inouye et al. disclose that the gene expression of the genetic material of cellular material or an organism can be regulated, inhibited or controlled by incorporating in or along with such genetic material, DNA or other genetic material which is complementary to the messenger RNA transcribed from the genetic material. In genetic antisense, a nucleic acid construct is added to the cell which causes the production in the cell of single-stranded nucleic acid that is antisense to the sense strands of the gene (or gene function) to be controlled, regulated, or inhibited.

In a different approach to Inouye et al., antisense oligos can be employed to control gene function. Antisense oligos are custom-synthesized short nucleic acids that complement a segment of a targeted sense strand. Antisense oligos have been disclosed in PCT Patent Application WO 83/01451, published on April 23, 1983, and also by Simons et al., "Translational Control of IS10 Transposition," Cell, 34:683-691 (1983). To control or regulate a targeted gene, antisense oligos must be added to the cell from the outside, much as a drug would be administered. In comparison to the longevity and relatively long-lasting effects afforded by genetic antisense constructs, antisense oligos generally provide transient (relatively short term) genetic regulation, and thus, must be reintroduced into the cells to maintain regulation. The distinction between permanent and transient effects should not be relegated, however, to genetic antisense and antisense oligos, respectively. That is to say, there are circumstances in which genetic antisense may provide relatively short-lived or transient effects, as well as circumstances in which antisense oligos may provide long-term or permanent genetic effects.

In accordance with the present invention, there is provided a new and unexpected use for rendering a system immunologically compatible based upon genetic intervention techniques, including antisense and other nucleic acid technologies. The present invention is applicable to a number of applications - both human therapeutic and veterinarian - where it is highly desirable if not essential to the subject's biological function that the immune response (Ir) gene(s) be inhibited or regulated. Such applications include the use in the treatment for autoimmune diseases, organ and tissue transplantation, and blood transfusions. Organ transplantation, for example, includes both allogeneic and xenogeneic transplantation the former referring to transplants between genetically different individuals within a species, whereas the latter refers to different individuals from different species. Because of the extreme risk of organ and tissue rejection in xenogeneic transplantations, the present invention will be particularly useful in such situations by overcoming or reducing the risk of rejection, while at the same time, providing advantages over the prior art transplantation methodologies, especially those related to immunosuppression, e.g., radiation and chemical agents or cytotoxic agents. In the present method, a system can be usefully rendered immunologically compatible for transplant and transfusion applications, as well as for treatment of autoimmune diseases. Such a system is rendered immunologically compatible by introducing into the cells of the system a nucleic acid construct which, when present in a cell, inhibits or regulates the function of an immune response (Ir) gene concerned with the determination of immunological determinants of the system, i.e., controlling the immune response.

As used in the present disclosure, the phrase "immunologically compatible system" refers to at least one member selected from the group consisting of an non-human organism, an organ, a tissue, or cells, which members have been rendered immunologically compatible. Immunological compatibility is rendered by introducing a gene-regulating nucleic acid construct into the cells of the system, the nucleic acid construct being based upon the principles and application of antisense technology. The term "immunologically compatible" is intended to encompass all aspects of immunology and molecular biologiy in which genetic intervention, namely antisense technology, is usefully employed to modify, alterate, regulate, suppress, or inhibit the immune state of the system or subject.

The term "immunological compatibility" is also intended to encompass the narrower state of immunologic tolerance in which the immune system is manifested by a diminished or absent capacity to express either cell-mediated or humoral immunity with respect to one or more specific antigen determinants. See, e.g., Immunology: A Short Course, Benjamini and Leskowitz, *supra.*

Immunological compatibility in any system can be measured or evaluated using methods known in the art. More particularly, various assays are available which can be used as a means for determining immunological compatibility. Many such assays are well described in Basic and Clinical Immunology, Stites and Terr, *supra.* These include carrying out the mixed lymphocyte culture (MLC), a cytotoxicity assay, and the detection of antibodies to donor antigens. It is possible, of course, to carry out simultaneous assays in order to "average" the results.

In preferred embodiments of the present invention, such an non-human organism, organ, tissue, or cells may be of mammalian origin, e.g., porcine, bovine, and may also be of simian origin.

It will be appreciated by those skilled in the art that immunological compatibility is largely dependent on the genetically-based immunological determinants of any biological system. Such immunological determinants are largely under the control of the class of genes known as the immune response (Ir) genes. Immune response genes are known to control the immune response of a system or organism to specific antigens. See, e.g., Basic and Clinical Immunology, Stites and Terr; Basic Human Immunology, Stites and Terr; and Immunology: A Short Course, Benjamini and Leskowitz, *supra.*

Most of the immune response genes are known to fall within the major histocompatibility complex (MHC), although this designation can also be used to described other types of genes which are outside the MHC. For example, proteins belonging to the MLS antigens (Minor Leukocyte Stimulating) can elicit an immune response. The MLS antigens are incorporated retroviruses which express proteins on cell surfaces which can activate and serve as targets for T cells. In other words, certain immune response provoking antigens are coded by retroviruses whose genetic information has been incorporated into the host genome. In addition, both the genes which are responsible for the classic ABO blood group system and other genes which produce the Rhesus blood group system may also be considered immune response genes in accordance with the present invention. The former group, the ABO system remains the most important blood group system for transfusion purposes. According to Donegan and Bossom ("Blood Banking & Immunohematology," Chapter 20 in Basic and Clinical Immunology, Appleton & Lange, *supra),* more than 400 erythrocyte antigens belonging to 24 systems are known today. Each blood group system has members, and each member may be composed of one or more different antigens. Each antigen is controlled by one gene. The ABO blood groups are determined by allelic genes, A, B and O. The antigenic determinants of a blood group are produced either directly (for proteins) or indirectly (for carbohydrates) by alleles at a single gene locus or at a gene locus so closely linked to another the crossing over is extremely rare. Furthermore, for any antigen of a group, a single allele is present at that locus and other are therefore excluded.

Classified as erythrocyte antigens are the carbohydrate moieties whose specificity resides in the terminal sugars of an oligosaccharide. Genetic control is effected through the H gene which codes for the production of transferase enzymes that conjugate terminal sugars to a stem carbohydrate. One such enzyme is fucosyl transferase that effects the addition of fucose to precursor chains and completes the stem chain. As discussed by Donegan and Bossom, the last three sugars of the stem chain are called H substance, and the Bombay phenotype in which the H gene (hh) is absent, is rarely found. Without a complete stem chain, additional sugars cannot be added despite any A or B transferase. As a result, high-titer anti-H is produced.

Second in importance to the ABO blood group system is the Rhesus (Rh) blood group system, the genes of which control more than 40 antigens.

Other minor genetically-based blood systems are also known and have been characterized. These include the Lewis and Secretor. Secretion of ABH substances in body fluids (saliva, sweat, milk, etc.) is controlled by allelic genes Se and se. These genes are independent of ABO.

Other erythrocyte antigens have been characterized, and these include about 20 blood groups, representing more than 300 antigens. Although rarely implicated in transfusion reactions, the Kidd, Duffy, Kell and MNS systems are known to cause hemolysis if antigen-positive blood is transfused into a sensitized recipient. All of the aforementioned blood group system are encompassed by the term "immune response gene" as used herein.

The histocompatibility determinants in humans are largely and genetically derived from the MHC, which is a cluster of genes occupying a region located on the sixth chromosome. Also referred to as the Human Leukocyte Antigen (HLA), the MHC has been divided into six major gene loci, designated as HLA-A, HLA-B, HLA-C, HLA-DP, HLA-DQ and HLA-DR. In general, the A, B and C loci encode the classical transplantation antigens, whereas the DP, DQ and DR loci encode products that control immune responsiveness. The MHC is further broken down into classes of antigens. For example, the Class I antigens include the histocompatibility antigens encoded in humans by A, B and C loci; in mice, such antigens are encoded by D and K loci. The Class II antigens include histocompatibility antigens encoded in humans by DR, DP and DQ loci. The Class III antigens are represented by C4 and factor B, which are complement components encoded within the MHC. Details concerning the biology and genetics of HLA antigens can be found in European Patent Appl. Publ. No. 0 103 960. Other information concerning the isolation and characterization of human HLA DNA antigens can be found in European Patent Appl. Publ. No. 0 439 208 (A2), published on July 31, 1991. The role of the MHC in the immune response is also discussed in Chapter 11 in Immunology: A Short Course (Benjamini and Leskowitz), *supra.*

Another source of information relating to the characterization of HLA Class II antigens particularly on a molecular level is the book edited by Jack Silver, Ph.D [Molecular Biology of HLA Class II Antigens, CRC Press, Inc., Boca Raton, FLA, 1990]. A number of articles are presented in this book including some dealing with the analysis of class II diversity using for example, cellular reagents, *trans-*complementation of DQ∂ and DQβ chains and mixed isotope diversity. In addition, the transfection of T-cell clones and L cells with class II cDNA in order to study antigen presentation function of class II genes is also disclosed in a chapter by Wilkinson. Other methods of analyzing class II diversity is disclosed in other chapters of this book, including DNA sequencing and allele specific oligonucleotide typing of PCR amplified DNA.

Another useful book on the subject of HLA Class II genes and antigens is The HLA System: A New Approach, edited by John Lee, Springer-Verlag, New York. Included among its useful topics are articles dealing with HLA-D region mapping, expression and regulation of hybrid Class II molecules, isotypic diversity and function of Class II antigens, analysis of Class II products by gene transfer, and the involvement of Class II genes in disease susceptibility, including normal immune responses and autoimmune diseases.

The Class I and Class II MHC antigens are actually protein(s) which express peptide derived from other proteins in the body. When these proteins are different from one member of the species to another, the peptide presented may be different. Thus, minor histocompatibility antigens represent alleic differences in other proteins, for example, between donor and a potential recipient.

As used herein, the term "immune response gene(s)" is also intended to encompass other genes which are responsible for non-antigen specific immune responses, including interactions involving cell adhesion molecules (CAMs). In the early stages of an immune response, circulating white blood cells, such as neutrophils and monocytes, classically migrate from blood vessels to sites of tissue damage in response to the presence of antigen. The migration and accumulation of white blood cells in the tissue leads to the acute inflammation associated with the following eventsreperfusion injury, septic shock, rheumatoid arthritis and atherosclerosis.

The release of cytokines, such as interleukin 1 (IL-1), from damaged or infected cells initiates the recruitment of neutrophils and monocytes which induce endothelial cells lining blood vessels to express the CAMs. The CAMs mediate the recruitment and subsequent migration of neutrophils and monocytes from the bloodstream to areas of tissue injury through the recognition of a specific ligand located on the surface of these cells in what could be termed a "lock and key" interaction. When the appropriate ligand or "key" fits the receptor or "lock" of the CAM, the gates which allow white blood cells to migrate from the bloodstream to the site of tissue injury are opened.

Among the classes of CAMs involved in acute or chronic inflammatory conditions are the so-called LEC-CAMS, integrins and Monocyte Cell Adhesion Molecules (MAMs). LEC-CAMs have been recently characterized and found to mediate binding and subsequent migration of neutrophils and monocytes during acute inflammation. Two members of LEC-CAMs are Endothelial Leukocyte Adhesion Molecule (ELAM-1) AND GRANULE MEMBRANE PROTEIN (GMP-140 or CD-62). The latter is usually expressed on the surface of endothelial cells immediately following tissue damage while ELAM-1 is expressed typically several hours later. The mediation of neutrophil and monocyte recruitment and the migration by both ELAM-1 and GMP-140 has been reported to be through recognition of the same carbohydrate molecule located on the surface of these white blood cells, known as Sialyl Lewis X(SLe^{x)}.

Integrins represent another class of CAMs that mediate recruitment and migration of white blood cells known as lymphocytes to sites of tissue injury in chronic inflammatory diseases. One integrin, known as VLA-4, has been implicated in lymphocyte migration in chronic inflammatory conditions through the recognition of a portion of the extracellular matrix protein fibronectin, known as CS-1.

The Monocyte Adhesion Molecules (MAMs) may play a significant role in the onset of atherosclerosis, on the basis that the binding of monocytes to arterial walls results in the formation of plaque which causes atherosclerosis.

Many of the above-described genes have been published and are readily available to the skilled artisan, including in electronic format. The National Center for Biotechnology Information (NCBI) (National Library of Medicine, Building 38A, NIH, 8600 Rockville Pike, Bethesda, MD 20894) produces CD-ROMs available by subscription through the Government Printing Office (GPO). NCBI-Genbank (Flat File Format) is a release version of GenBank^{®} data supplemented by new data from direct submissions, the NCBI Backbone (journal scanning), and EMBL and DDBJ DNA databases. Also available through NCBI is Entrez Sequences which contains an integrated view of DNA and protein sequences and their associated MEDLINE entries. DNA and protein sequence data can be retrieved; non-redundantly, from GenBank, NCBI Backbone, EMBL, DDBJ, SWISS-PROT, and PIR and are linked to journal citations appearing in MEDLINE^{®}. In addition, other published sequences are available from Intelligenetics, Inc. (700 East El Camino Real, Mountain View, CA 94040).

As described hereinabove and in further detail below, the nucleic acid construct used in accordance with the present invention can be usefully employed both in the form of genetic antisense and antisense oligos.

When in the form of genetic antisense, the nucleic acid construct contains a nucleic acid segment that produces a nucleic acid sequence that is complementary to at least a portion of a ribonucleotide sequence transcribed from the immune response gene that is responsible for controlling the immune response to specific antigens, and more particularly, for example, the histocompatibility determinants of any individual. By producing such a nucleic acid sequence, the nucleic acid segment contained in the nucleic acid construct effectively inhibits, regulates, inhibits, suppresses, or modifies the function of an immune response gene in any given system.

In other aspects of the present invention, the nucleic acid segment contained in the nucleic acid construct may include particular regions of an immune response gene in a system, including, for example, the 5' non-coding region of the gene, the ribosome binding portion of the gene, or the translation initiation portion of the gene.

The construction or synthesis of the nucleic acid constructs useful in accordance with the present invention is described in the aforementioned Inouye et al. patent application disclosures.

In the practice of the present invention, the nucleic acid construct may further comprise a transcriptional promoter segment, and a transcription termination segment, with the nucleic acid segment, above-described, located between the just mentioned promoter and termination segments. Further described is that the transcriptional promoter segment may comprise an inducible promoter.

When employed in.the form of antisense oligos, the nucleic acid construct generally comprises a modified oligonucleotide complementary to at least a portion of the ribonucleotide sequence transcribed from a given immune response gene. It will be appreciated by those skilled in the art that in accordance with the present invention, the nucleic acid construct comprises a plurality of modified oligonucleotides complementary to different portions of the ribonucleotide sequence transcribed from the immune response gene or genes responsible for the genetic or immunohisto-compatibility determinants, as defined above.

When modified, the antisense oligos may comprise a number of forms, including the following modified member an association constant increasing modifier, an association increasing modifier, or a combination of any of the foregoing. Such modifying members are known in the art, having been disclosed in European Patent Appl. Publ. No. 0 450 370 A1, published on October 9, 1991, based upon the priority document of Wetmur et al., U.S. Patent Appl. Ser. No. 07/499,938, filed on March 26, 1990. In addition, modifications involving the use of intercalators are described in European Patent Appl. Publ. No. 0 231 495 A2, published on August 12, 1987, based upon Stavrianopoulos et al., U.S. Patent Appl. Ser. No. 07/394,284, filed on August 15, 1989, which is a continuation of Ser. No. 06/808,757, the latter filed on December 13, 1985.

The invention at hand is also concerned with the use for rendering a system immunologically compatible, which use involves the introduction of a nucleic acid construct into cells, which can be mammalian. Among appropriate cells for introducing the nucleic acid construct are non-human germ cells, fertilized cells, non-human embryonic cells and somatic cells. After its introduction and when present in a cell, the nucleic acid construct inhibits or regulates the function of an immune response gene in the system. Thereafter, the thus-modified cells can be allowed to develop into a mature immunologically compatible system, which can serve as a source of immunologically compatible organs, tissues and cells for human transplant recipients. The term "transgenic" is applied to mature animals having germ cells and somatic cells containing a gene introduced into the animal (or an ancestor of the animal) at an embryonic stage. Leder et al., U.S. Patent No. 4,736,866 disclose a transgenic non-human eukaryotic rodent animal containing an activated oncogene (myc) that was introduced by injection into the male pronucleus of fertilized mouse eggs, and later transferred into pseudopregnant foster females for development to term. Leder et al. disclose various other methods for introducing a gene (oncogene) into an animal embryo for chromosomal incorporation in an activated state. One way is to transfect the embryo with the gene as it naturally occurs, and then to select transgenic animals in which the gene has integrated into the chromosome at a locus which results in activation. Another way is to modify the gene (sequence) or its control sequences prior to its introduction into the embryo. In this regard, the embryo can be transfected using a vector, e.g., plasmid, containing an already translocated gene. Other methods may include the use of a gene whose transcription is controlled by a synthetic or viral activating promoter, or a gene activated by one or more base pair substitutions, deletions, or additions. See Leder et al., U.S. Patent No. 4,736,866, e.g., column 1, lines 42-56.

Transfection of the nucleic acid construct described herein can also be used on organisms, including non-human mammals and simians, at the non-embryonic stage of development. Dubensky et al. ("Direct transfection of viral and plasmid DNA into the liver or spleen of mice," Proc. Natl. Acad. Sci. (USA), 817529-7533 (1984)) disclose, for example, a method for the direct transfection of polyoma viral DNA and polyoma-plasmid recombinant DNA into the liver or spleen of newborn or adult mice. In their disclosed method, Dubensky et al. injected calcium phosphate-precipitated DNA directly into mouse organs in combination with hyaluronidase and collagenase. The transfected DNA was shown to replicate at moderate efficiency, relative to the direct infection of organs with virus (Hepatitis B virus), the latter leading to acute infection. A polyoma-bacterial plasmid recombinant DNA also was shown to replicate when transfected into mice. Thus, Dubensky's disclosed method allows the direct determination of the biological activity of a cloned DNA within a mouse organ, and can likewise be used in the present invention to determine the biological activity of the nucleic acid construct, described above, after its introduction into cells.

The principles of genetic material transfer employed by Leder et al. to develop a transgenic non-human mammal are applicable to the present invention. Non-human transgenic mammals, including transgenic simian organisms, can be developed using the method(s) disclosed in Leder et al. See also the references cited in U.S. Patent No. 4,736,866, e.g., Palmiter et al. ("Differential Regulation of Metallothionein Thymidine Kinase Fusion Genes in Transgenic Mice and Their Offspring," Cell, 29 701-710 (1982)).

The introduction of the presently described nucleic acid construct into the cell(s) can be used in various ways known to those skilled in the art. For example, a vector having the nucleic acid construct incorporated into it can be used to introduce the construct into the cell(s), or the embryonic non-human mammalian cells. Such vectors are well described in the art and may take the form of a plasmid, or a recombinant virus. These are well known in the art. See, e.g., Maniatis et al., Molecular CloningA Laboratory Manual, Cold Spring Harbor Laboratory, 2nd edition, 1989.

The quantity of the nucleic acid construct required to inhibit or regulate the function of an immune response gene or genes in a system does not require precise quantitative values particularly such values may be inappropriate, much in the same way that antibody-antigen interactions do not require precise values in order to observe the manifestation of a response. One reason why such precise values are not required to describe antibody-antigen interactions stems from the fact that such binding interactions are relatively weak. For another, it may very well be that an antibody produces an appropriate immune response, for example, by bringing the presentation of antigens below a threshhold level, say 40% for the sake of argument, and only the antibody-antigen interactions above that number are significant for effecting a change in the immune response. In the same way, a nucleic acid construct that has been introduced into the cells of a system to attain immunological compatibility in the system, may be effective in bringing the number of antigens below the threshhold level required for provoking an immune response. Thus, the quantity of the nucleic acid construct that should be introduced into the cells of the system as described herein should be sufficient to inhibit or regulate the immune response gene, the physiological and biological manifestations of which can be measured and assessed by conventional methodology.

In the case of organ and tissue transplantation, monitoring can be carried out by methods well known in the art, including for example, needle biopsy, and fine needle aspiration. For a discussion of the latter technique, see Hayry et al., "Fine Needle Aspiration in Transplantation Pathology," Chapter 12, in The Pathology of Organ Transplantation, *supra.*

### Examples

### Organ, Tissue and Cell Transplantation Application

### 1. Isolation of Class I MHC genes.

Cytotoxic T lymphocytes require the presence of MHC Class antigens on target cells for adhesion as well as for triggering of the antigen-specific T cell receptor. The presentation of Class I MHC antigens on the surfaces of most somatic cells is the primary underlying factor responsible for the xenogeneic response that results in the rejection of transplants and presumably in autoimmune rejection of tissues. Faustman et al., *supra,* have demonstrated that antibody masking of the MHC class I antigen expressed on the surface of pancreatic islet cells can be used to prolong the acceptance of transplanted islet cells in mice. Antigen masking is likely to be practicable for only the introduction of monodisperse cells into a recipient animal, particularly because its feasibility for use in preparation of organs for transplant into recipient xenogeneic animals remains to be demonstrated.

A more productive method for the preparation of "universal donor organs" for use in temporary and/or permanent transplant situations might be the use of antisense inhibition of the MHC genes in specially designed donor animals. Such transgenic or chimeric animals, for example, goats, pigs, sheep, baboons and chimpanzees or other nonhuman primates, might be bred and raised as organ donors for use in organ transplants in humans in situations in which human donors are either temporarily or permanently unavailable. Other situations in which the antisense inhibition of MHC genes might be used for transplantation are in cultured organs, particularly in cultured skin. The antisense inhibition of MHC genes might find a ready application in bone marrow transplant. When bone marrow is isolated from a human (or possibly non-human) donor, and expanded in culture before transplant, it is a simple matter to introduce the antisense-MHC genes into the cells by various gene-transfer methods, including infection with recombinant retroviruses. In some cases, perhaps particularly when an adequate match has not been possible, the re-population of the patient may be better achieved with such a bone marrow.

In order to carry out such a method, a primary requisite would be the appropriate isolated MHC or other histocompatibility-determining genes. Moore, et al. ("DNA Sequence of a Gene Encoding a BALB/c Mouse Ld Transplantation Antigen," Science 215:679-682 (1982)), Goodenow et al., Science 215:677-679 (1982)), Singer, et al. ("Characterization of a Porcine Genomic Clone Encoding a Major Histocompatibility AntigenExpression in Mouse L Cells," Proc. Natl. Acad. Sci. 79:1403 (1982)), and Barbosa, et al. ("Identification of Human Genomic Clones Coding the Major Histocompatibility Antigens HLA-A2 and HLA-B7 by DNA-Mediated Gene Transfer," Proc. Natl. Acad, Sci. 79:6327-6331 (1982)) describe the isolation and partial characterization of murine, porcine, and human HLA class I genes that would be appropriate for the proposed uses. In these examples, the DNA of the organism in question was obtained, digested with an appropriate restriction endonuclease into fragments of an appropriate size and then cloned into a bacteriophage lambda vector forming what is known as a genomic DNA library. Alternative procedures involve the production and cloning of cDNA libraries using mRNA as the starting material. Such methodologies are straightforward and known to those skilled in the art as described in Sambrook et al. (Molecular Cloning: A Laboratory Manual (2nd edition, Cold Spring Harbor Laboratory Press, New York (1989)). Such cDNA libraries can be screened by the DNA hybridization methodologies described in Singer et al., *supra,* or, perhaps more easily and specifically, when prepared as cDNA libraries in appropriate vectors (i.e, expression vectors as lambda gt 11), they can be screened by immunological techniques for the production of the particularly desired MHC antigen as described in Sambrook, et al., *supra,* and other method reference sources. While there are some advantages to the isolation of the genomic clone for the MHC gene of interest, an isolated cDNA clone can be used straightforwardly to screen a recombinant library for genomic clones of the same and related genes.

One of the advantages of the genomic clones is that, at least according to some workers in the field of antisense technology, the intro/exon junctions and the non-coding regions of genomic clones may be more effective in generating effective antisense RNA molecules (see, for example, Strickland et al., "Antisense RNA Directed Against the 3'-Noncoding Region Prevents Dormant mRNA Activation in Mouse Oocytes," Science 241:680-684 (1988); and Ch'ng et al., Antisense RNA Complementary to 3' Coding and Noncoding Sequences of Creatine Kinase is a Potent Inhibitor of Translation in vivo," Proc. Natl. Acad. Sci. 86:10006-10010 (1989)). One disadvantage of the genomic clone is that the cDNA clone, or minigene as it is sometimes referred, is not obtainable in a straightforward manner from the genomic clone, although the genomic clone (or a portion thereof) might be used as a gene-specific probe of cDNA clones in a cDNA library. The other disadvantage of isolating only the genomic clone of MHC genes is that the use of some vectors, particularly retroviral vectors is not applicable to DNAs which are too large. Retroviral vectors are useful for introduction of foreign genes into such tissues as blood and bone marrow and cannot accommodate extremely large fragments of DNA.

Alternatively, other genes whose gene products are required for MHC presentation might be the targets of antisense inhibition. For example, β₂ microglobulin and/or adhesin molecules are required for proper MHC presentation. The inhibition of expression of these genes by antisense genes might be an effective way to inhibit MHC presentation. However, one might anticipate that inhibition of these genes may have more substantial adverse consequences for the viability and health of the animal in question. Adhesin molecules are probably not required for Class I expression but they are required, however, for proper functioning. Peptide transporters are required for expression along with β₂ microglobulin.

In order to isolate the murine cDNA clone for the murine L^{d} transplantation antigen, the cDNA library of a C57 Black female mouse heart is prepared (or purchased from Stratagene Cloning Systems, La Jolla, CA, or Charles River Breeding Laboratories, Wilminton, MA). The lambda ZAP^{®} vector into which the DNA is cloned allows for the expression of a b-galactosidase fusion proteins from the inserted cDNAs. Thus, appropriate phage growths (see reference 21) can be screened for the production of β-galactosidase-MHC L^{d} fusion protein using antibodies described in Goodenow et al., *supra,* and also methods described in the Sambrook, et al., *supra.* Alternatively, the individual lambda clones can be screened using the MHC probes described in Steinmetz, et al. (Cell 25:683 (1981)).

Alternatively, blood or spleen or other tissue from a C57 Black mouse can be used as the source of RNA for the production of a new cDNA library. Methods described in Sambrook et al., *supra,* are useful for purifying poly A containing RNA from homogenized tissue or grom lymphocytes isolated from blood. Briefly, RNA can be prepared from cells by the guanidine thiocyanate-Cs-trifluoroacetic acid method as described by Okayama, et al. ("High-Efficiency Cloning of Full-Length cDNA Construction and Screening of cDNA Expression Libraries for Mammalian Cells," Methods in Enzymology 154:3-28 (1987)), and the RNA can be converted to double-stranded cDNA by random hexanucleotide-primed reverse transcription using an Amersham cDNA Synthesis kit (Amersham International, Amersham, U.K.). Synthetic adaptors, e.g., Eco RI or Bam HI adaptors, are ligated to the cDNA using T4 DNA ligase, and the material is then phosphorylated with T4 kinase. About 100-200 ng of the adaptor-ligated, phosphorylated cDNA is next ligated to approximately 1 µg of lambda gt 11 phage vector arms. The ligated DNA is then packaged *in vitro* using a commercial packaging kit, e.g., Gigapack Gold (Stratagene Cloning Systems, La Jolla, CA). Packaged particles are then plaque titered and plaques are screened for MHC expression using antibodies against the MHC L^{d} protein (see, e.g., Goodenow et al., *supra).*

Alternatively, the individual plaques are blotted onto filter paper and screened for complementarity to the MHC sequences (Moore et al., *supra)* by DNA hybridization methods. The appropriate cDNA clone(s) is/are then picked and expanded in an appropriate bacterial host, and characterized by restriction mapping. This is most readily accomplished by removing the inserted cDNA from the lambda vector by Eco RI digestion (or Bam HI digestion, depending upon the adaptor used above) and cloning the DNA into a plasmid vector such as the pUC19 or pIBI31.

The expanded recombinant plasmid DNA is then characterized by dideoxynucleotide sequencing and by restriction enzyme analysis. The sequences is compared to known sequences of previously isolated MHC genes (e.g., Moore et al., *supra*), and the sequence of the 5'-untranslated and 3'-non-coding regions and polyadenylation sites in the sequence are determined.

Other genes of importance, both genomic and cDNA genes can be isolated and characterized by the same or similar techniques. In fact, prepared cDNA libraries and genomic DNA libraries in lambda ZAP^{®} II (Stratagene Cloning Systems, La Jolla, CA) can be screened directly as described above, provided the appropriate antibodies (e.g., anti-mouse adhesin) and/or DNA probes are available.

### 2. Derivation of antisense Class I MHC or HLA expression constructs.

Lotteau, et al. ("Modulation of HLA Class II Antigen Expression by Transfection of Sense and Antisense DRalpha cDNA," J. Exp. Med. 169:351-356 (1989)), Weinberg et al.("Methods and Artificial Genes for Antagonizing the Function of an Oncogene," U.S. Patent No. 4,740,463), Gunning et al. ("A Human β-actin Expression Vector System Directs High-Level Accumulation of Antisense Transcripts," Proc. Natl. Acad. Sci. 84:4831-4835 (1987)) and Von Ruden et al. ("Inhibition of Human T-Cell Leukemia Virus Type I Replication in Primary Human T Cells that Express Antisense RNA," J. Virol. 63:677-682 (1989)) discuss the design and preparation of various "antisense" RNA constructs for use in the suppression of expression for particular genes. While the discussion in these articles is directed toward the gene of interest in each particular study, the required feature of all antisense constructs are the three major components required for the construction of any artificial gene for its introduction into mammalian cells. The requirements are, first, a promoter to be recognized by RNA polymerases within the cell. Secondly, the construct requires the gene or the reverse (antisense) gene (or portion thereof) of interest. Lastly, the constructs generally require 3' non-coding sequences that allow poly-adenylation of the RNA transcript.

Many of the constructs in the referenced works employ the mouse metallothionein promoter, the CMV immediate early promoter, or the promoter found in retroviral long terminal repeats (LTRs). Other promoters, particularly those which could be inducible by administration of a specific hormone, might be especially useful for the application of the antisense MHC genes. In accordance with the present invention, it is useful to employ sequences which may be located immediately upstream of the MHC gene of interest. Maguire et al. ("Regulation of Expression of a Class I Major Histocompatibility Complex Transgene," in Genetic Engineering of Animals, W. Hansel and B. J. Weir eds., The Dorset Press, Dorchester, ENG, 1990, pp. 59-62; see also J. Reprod. Fert. Supplement, 41:59-62)) and Kimura et al. ("Detailed Analysis of the Mouse H-2Kb PromoterEnhancer-like Sequences and Their Role in the Regulation of Class I Gene Expression," Cell 44:261-272 (1 986)) indicate that upstream of the coding regions of porcine and murine MHC genes, there are promoter, enhancer-like sequences that may be valuable for maintaining tissue specific expression of the MHC gene and, therefore, they could be used to assist in generating tissue-specific expression of the MHC antisense gene of interest. Therefore, it might be useful to isolate and characterize the upstream regions when genomic clones of a particular MHC gene are isolated. The probable success of such an approach is also suggested by the use of the basic myelin gene promoter as the promoter for the basic myelin antisense gene in the production of the shiverer mice (Katsuki et al., "Conversion of Normal Behavior to Shiverer by Myelin Basic Protein Antisense cDNA in Transgenic Mice," Science 241:593-595 (1988)).

Many of the above referenced articles utilize the reverse-oriented cDNA (minigene) copy of the mRNA for the antisense construct. The optimal content of the antisense construct between the promoter and the poly adenylation signal sequences must be determined emprirically (see below). Portions of genes, intron/exon junctions, 5' and 3' non-coding regions all have been used successfully in creating antisense constructs. In addition, the use of ribozymes to further enhance the activity of a particular antisense sequence might be advantageous (Cameron et al., "Specific Gene Suppression by Engineered Ribozymes in Monkey Cells," Proc. Natl. Acad. Sci. 86:9139-9143 (1989); and Chang et al., "Ribozyme-Mediated Site-Specific Cleavage of the HIV-I Genome," Clinical Biotechnology 2: 23-31 (1990)).

The requirements for the 3' end of antisense genes has not been well studied, although most if not nearly all antisense constructs that have been been used in higher organisms (i.e., organisms having poly A sequences on their mRNAs) have utilized a signal for poly adenylation of the antisense transcript. The necessity and/or efficacy of the addition to the antisense RNA has not been adequately examined. It might be preferable to replace the poly A addition signal and the poly A sequence with, for example, a tRNA sequence or some other structural type sequence that, presumably like poly A, offers the remainder of the RNA some protection from digestion by 3'-specific ribonucleases.

The genomic murine MHC L^{d} clone described by Moore et al., *supra,* is cut into various pieces by restriction enzyme digestion. For example, Bam HI/Kpn I double digestion yields 2 Bam HI/Kpn I fragments from the genomic MHC L^{d} DNA, as well as a Bam HI/Bam HI fragment. Bam HI/Pst I double digest yields, as one of its five (5) resultant fragments, a Bam HI/Pst I subfragment piece of the Bam HI/Bam HI fragment from the Bam HI/Kpn I digestion. Each of these fragments can then be subcloned into a convenient vector, e.g., pUC19 or pIBI 31 for purpose of expanding the DNA. These DNA clones are designated pMHC L^{d}BK, pMHC L^{d}KB, and pMHC L^{d} BP and have the following contents

pMHC L^{d} BK--The Bam HI-Kpn I fragment from the 5' end of the genomic gene copy, containing exons 1 and 2 and intron 1 and a portion of intron 2;

pMHC L^{d} KB-the Kpn I-Bam HI fragment from the central portion of the genomic gene copy, containing exon 3 and a portion of intron 2 and most of intron 3; and

pMHC L^{d} BP--the Bam HI to Pst I fragment from the 3' portion of the genomic gene copy containing exons 4,5,6,7 and 8 and introns 4, 5, 6 and 7, a portion of intron 3 and some intergenic DNA.

Each of these clones is then modified to contain two terminal Bam HI sites by addition of adaptors (linkers), a Kpn I/Bam HI linker for both pMHC L^{d}BK and pMHC L^{d}KB, and a Pst I/Bam HI linker for pMHC L^{d}BP. The designation of these clones for these purposes will be changed to pMHC L^{d} BKB, pMHC L^{d}KBB and pMHC L^{d} BPB to denote the initial cut as well as the addition of the extra Bam HI site in each subject piece of DNA.

Thus, with this modification, each of the DNAs can then be inserted into the Bam HI site of pHβ APr-1-neo (Gunning et al., *supra)* in both the sense and antisense direction. By restriction enzyme digestion, the orientation of the clones for each of the genomic subclones can be determined. Each of the clones can then be examined for its ability to express the transcript after transfection, electroporation or other transfer to mouse tissue culture cells (see below).

Similarly, the cDNA clone from Example 1 above can be recloned into pHβ APr-1-neo (Gunning et al., *supra)* in both orientations and the sense and antisense clones can be separately isolated and tested. Subfragments of the cDNA clone, which can be prepared by restriction digestion and recloning, can also be recloned in one or both orientations into pHb APr-1-neo (Gunning et al., *supra).*

Once these DNA fragments are obtained, it would also be appropriate to clone the fragments and the entire cDNA sequence into retroviral vectors, such as those described by Von Ruden et al., *supra*). Methods for their re-cloning are well known to those skilled in the art. See, e.g., Molecular Cloning: A Laboratory Manual, Maniatis et al., 1989, *supra.*

### 3. Inhibition of MHC presentation by antisense, transformation.

The above constructs obtained in the previous example can be introduced by DNA transfection (e.g. classic transfection using CaPO₄ or by some other conventional means, such as lipojection or electroporation) and/or by infection with a retrovirus into cultured murine cells. The construct that produces the most efficient inhibition of MHC presentation will be selected for use in the production of a "transgenic" or chimeric mouse that is MHC-negative (see Example 4 below) and for introduction into the bone marrow and blood stem cells of a mouse (see Example 5 below).

Methods for transfection and for infection of cultured cells with retroviral vectors are known in the art, as found in various articles referred to above (see, e.g., Gilboa et al., J. Virol., 50:417 (1984)). Selection of transformed cells by neomycin (G418) selection is also described in any of the aforementioned articles and handbooks.

Analysis of the efficacy of each of the clones for inhibition of MHC synthesis can be carried out by comparing MHC levels in the cell lines that were transfected (or infected) with the sense versus the antisense orientations of the genes. For these experiments, it is useful that one initially chooses a cell culture line that ordinarily expresses the MHC product of interest. As an alternative to this approach, one can obtain the transformed murine cells that specifically express MHC L^{d} (see Goodenow et al., *supra)* from Dr. Leroy Hood's laboratory. These cells should be tested for MHC presentation after re-transfection (or infection) with the various antisense constructs. Similarly, any histocompatibility gene can be initially isolated and transfected into a cultured cell line to make an appropriate expressing cell line for testing various antisense constructs of the same gene, much in the manner that Goodenow et al., *supra,* prepared a murine line that specifically expresses HLA L^{d} and Korman et al. ("Expression of Human Class II Major Histocompatibility Complex Antigens Using Retrovirus Vectors," Proc. Natl. Acad. Sci. 84:2150-2154 (1987)) prepared a mouse cell that expresses human class II MHC antigens. The analysis of each of the antisense clones should involve not only testing for the expression or inhibition of expression of the targeted MHC molecule, the level of expression and the stability of the antisense RNA should be evaluated by conventional means as well.

The gene construct that provides the most significant inhibition of the MHC target gene and makes the most stable and abundant antisense transcript can then be used in the preparation of transgenic non-human animals.

### 4. Production of an HLA-negative mouse.

The methods described in Katsuki, et al., *supra,* can be used to introduce anti-MHC genes into "transgenic" mice.

C57 Black mice should be made genetically HLA L^{d} negative by introduction of one or another of the antisense constructs described above in Example 2. The mice should be expected to be immunologically suppressed and quite susceptible to disease, etc. Therefore, special precautions, such as those appropriate for nude and SCIDS mice, would be required. The level of expression of the HLA L^{d} gene product in various tissue and cells could be evaluated by appropriate immunohistochemical techniques.

Methods for introduction of antisense MHC genes into other organisms, such as pigs, goats, cattle and non-human primates are described in various of the publications discussed above.

### 5. Testing of MHC L^{d}-negative organs.

Testing of the transgenic, MHC L^{d}-negative mouse organs could be accomplished in a manner similar to that used by Faustman et al., *supra,* for testing the efficacy of MHC masking. Fluorescence-activated cell sorter (FACS) analysis can be employed to monitor and determine successful transplantation. FACS analysis involves staining a recipient's cells with different monoclonal antibodies to determine the type or nature of the cells, including the presence or absence of NK (natural killer) cells or CTL (cytotoxic T-lymphocytes).

### 6. MHC negative bone marrow.

Appropriate if not preferred for use in this transformation would be a retroviral anti-MHC construct. The retroviral construct that meets the criteria described in Example 3 above should be tested for use in mouse bone marrow.

Testing of various MHC-negative bone marrows would be accomplished by introduction of the modified bone marrow into irradiated animals and examining the viability of the blood and its ability to repopulate the bone marrow of the recipient animal. Preferably, this would be a trans-species study, i.e., MHC-negative canine bone marrow would be introduced into irradiated sheep (or goats or pigs) and the survival of the canine blood would be examined in comparison to the survival of MHC-positive canine bone marrow.

### 7. MHC-negative Human organs in culture--cultured skin

The human MHC genes described in Barbosa et al., *supra,* can be manipulated in a manner similar to that described in Examples 1 and 2 above for the murine MHC L^{d} gene. Human HLA class I cDNAs can be isolated and manipulated and place in antisense expression vectors. These antisense constructs can be transfected (or infected using retroviruses) into human skin cells in culture. The skin in the culture can then be monitored by conventionally known methods for presentation of the antigen in question. Successful inhibition of the HLA presentation can then be examined for its effect on graft acceptance and rejection in volunteer human skin graft patients.

### Other Exemplification

Clone pMHC L^{d} BKB DNA (5µg) (see Example 2 above), containing the first and second exons of the MHC L^{d}C57 Black gene, is digested with 25 units of Bam HI (New England Biolabs, NEBL) for 60 minutes. The resultant digest is fractionated on a 1.8% low melting temperature agarose gel and the small (approximately 0.7 kb) DNA fragment is isolated. phβ APr-1-neo DNA (2µg) is next digested with Bam HI (NEBL) and the digestion is stopped by heating to 68°C for 1 5 minutes. One microgram of Bam HI cut pHβ APr-1-neo DNA is mixed with the isolated MHC L^{d} BKB fragment and the mixture is ligated T4 DNA ligase, NEBL) for 16 hours at 18°C. The resultant ligated DNA mixture is introduced into competent E. coli cells (e.g. DH 5 alpha) and colonies containing plasmid are selected using amplicillin as a marker. Colonies containing recombinant pHβ APr-1-neo are identified by DNA miniscreen procedures which are also used to identify colonies containing the sense and antisense orientations of the MHC L^{d} BKB fragment.

The sense (MHC L^{d}) and antisense (L^{d} ALH) DNAs are transfected into C57 Black mouse fibroblasts by the the method of Graham and Van der Eb (Virology 52:456 (1973) as modified by Anderson et al., Cell 16:63 (1979)). In short, 50-100 ng of each DNA is mixed with 37.5-75 µg of sonicated NIH 3T3 carrier DNA, ethanol precipitated, and resuspended in 1-2 ml of 8 g/L NaC1, 5 g/L HEPES (pH 6.95), and 0.15 g/L Na₂HPO₄ X 7H₂O. A precipitate is formed by adding 50 µl of 2.5M CaC1₂ with vortexing. The precipitation process is allowed to continue for 30 minutes at which time the C57 Black cells are fed with 5 mls of 10 percent calf serum/Dulbecco's Modified Eagle Medium (DMEM) and the DNA precipitate. Four to 7 hours later, the precipitate is removed and the cells are re-fed with 10 mls of DMEM, 10% calf serum. After 16 to 24 hours, the cells are split 1 to 4 and fed with DMEM medium containing 10% calf serum and 1 mg/ml geneticin (G418). Cells are then re-fed each day with G418-containing medium unitl only G41 8-resistant cells remain in the dishes, indicating cells which contain the introduced plasmid DNA. Individual colonies, i.e., clones, are then isolated using cloning rings or by limiting dilution into microplate culture wells.

Individual clones of cells transfected with either the sense, MHC L^{d} gene construct or the antisense, L^{d} ALH, gene construct are tested for the expression of L^{d} antigen by the method of Goodenow et al., *supra.* Briefly, 50 µl of a single cell suspension of transformants (1 X 10⁷ cells per ml in DMEM with 10% serum and 10mM HEPES) are mixed with 50 µl of dilutions of monoclonal hybridoma antibodies to Class I molecules in flexible microtiter plates (Dynatech). Cells are incubated for 1 hour at 20· C, washed twice and resuspended in phycoerythrin linked goat anti-mouse lg F(ab')². The cells are further incubated at 20· C for 45 minutes. The unbound conjugate is washed away and the cells fixed in 1 % paraformaldehyde. The presence of antigens can be determined by flow cytometry.

As an alternative method, after approximately 45 minutes of incubation at 4°C, the cells are washed twice with medium and resuspended in 50 µl of medium containing approximately 75 X 10³ cpm of ¹²⁵I-labeled protein A (Amersham). The cells are incubated overnight at 40·C and washed twice before counting in a Searle gamma counter. Isolated clones of cells which are transformed with the sense, MHC L^{d}, gene construct are expected to express normal to elevated amounts of L^{d} antigen, while the opposite is true for cells transfected with the antisense, L^{d} ALH, gene construct. Isolated clones of cells that are transformed with the antisense, L^{d} ALH, gene construct which fail to express normal levels of L^{d} antigen are then tested for the presence of the human Eβ-actin gene 5' flanking sequence, indicating the presense of the introduced antisense construct.

Of more recent vintage is the FACS machine (briefly described above) which can be used to sort and analyze cells. See, e.g., Hulett et al., "Development and Application of a Rapid Cell Sorter," Clin. Chem., 19:813 (1973).

The antisense cell clones that express very low levels of MHC L^{d} antigen are tested for the presense of MHC L^{d} anti-mRNA by RNA isolation and Northern blotting. These procedures are repeated for each of the MHC L^{d} constructs identified in Example 2 hereinabove. The antisense construct that produces the most significant reduction in MHC L^{d} expression is used for the production of C57 Black mice which fail to express the MHC L^{d} transplantation antigen.

The following describe various approaches which can be used to render allografts non-immunognic.

### Double Knockout

Organs and tissues lacking both class I and class II MHC antigens fail to elicit any T cell response. T cells respond to foreign peptides presented by self-antigens. Allogenic MHC antigens elicit a particularly vigorous response which probably represents a perception by immune T cells of such allogenic molecules as self with peptide. MHC on a foreign cell has the potential to elicit rejections by virtue of the presentation of foreign peptides. Therefore, using the genetic intervention concepts of the present invention, for example, appropriate nucleic acid constructs as described herein, the expression of both class I and class II MHC antigens in the donor strain can effectively be eliminated. Special facilities may be required for housing such animals because of susceptibility to infection. This susceptibility is due to the fact that cytotoxic T cells are believed to be primarily responsible for recovery from viral infections. T helper cells probably function to provide cytokines to cytotoxic T cells which are necessary for efficient functioning.

The antigen presenting cells of a recipient can function to scavenge microbes and present them to recipient T helper cells, but in order for cytotoxic T lymphocyte to eliminate virus from infected cells, a class I antigen must be expressed. In accordance with the present invention, upon elimination of the foreign MHC class I and class II molecules, a human gene coding for a class I MHC antigen compatible with the recipient, may be inserted, thus affording protection to retroviral and other related infections. By so doing, the virus can be eliminated from infected cells without eliciting organ or tissue graft rejection.

A third approach is to eliminate only the foreign class II antigens using the genetic intervention of the present invention. David Sachs, *supra,* has suggested that the response to donor-recipient disparities for class I antigens can be controlled effectively by immunosuppressive agents, such as cyclosporin, but that class II disparities cannot. In accordance with the present invention, genetic intervention, for example, in the form of nucleic acid constructs, can be usefully applied to eliminate class II genes on the donor organs, but leave the class I genes intact. Potential recipients receiving such transplants would probably need a regimen of immunosuppressive drugs but the drugs would not have to be administered for the remainder of the recipient's life.

### Autoimmune Disease Application

Autoimmune is arbitrarily divided into organ specific and non organ specific disease. Most disease is mediated by antibodies of the IgG class which are dependent on T cells for their maturation. Many theories of autoimmunity suggest that autoimmune disease results from the inappropriate expression of class II antigen on cells in organs such as the thyroid gland or the pancreas. Among the more notable examples of diseases where genetic intervention can be usefully applied to control or eliminate inappropriate expression of class II antigens are thyroid related autoimmunity, such as Graves disease or Hashimoto thyroiditis, rheumatoid arthritis, and even diabetes.

## Claims

1. A nucleic acid construct comprising a plurality of oligonucleotides complementary to different portions of a ribonucleotide sequence transcribed from an immune response gene in a system which construct when present in a cell, inhibits or regulates the function of said gene, wherein said immune response gene codes for (a) Class I or Class III antigens of the major histocompatibility complex, or (b) cell adhesion molecules, and wherein said complementary oligonucleotides comprise at least one modified member selected from the group consisting of an association constant increasing modifier and an association increasing modifier, or a combination of any of the foregoing.

2. The nucleic acid construct according to claim 1 further comprising a transcriptional promoter segment and a transcription termination segment.

3. An immunologically compatible system excluding the human body containing a nucleic acid construct according to claim 1 or 2.

4. The immunologically compatible system according to claim 3 comprising at least one member selected from the group consisting of an organism, an organ, a tissue, and cells, or a combination thereof.

5. The immunologically compatible system according to claim 4 wherein said organism, organ, tissue or cells are mammalian.

6. The immunologically compatible system according to 5 wherein said mammalian organism, organ, tissue, or cells are simian or porcine.

7. Use of a nucleic acid construct according to claim 1 or 2 for the preparation of a pharmaceutical composition for rendering a system immunologically compatible, whereby the composition is suitable for the introduction into the cells of said system.

8. The use according to claim 7 wherein said immunologically compatible system is defined according to any one of claims 4 to 6.

9. The use according to claim 8 wherein said introducing step is to be carried out with a vector having incorporated therein said nucleic acid construct.

10. The use according to claim 9 wherein said vector is a plasmid or a virus.

11. The use according to claim 7 wherein said introducing step is to be carried out with a delivery system for delivering said nucleic acid construct to said cell.

12. Use of a nucleic acid construct as defined in claim 1 or 2 for the preparation of a pharmaceutical composition for rendering a system immunologically compatible, whereby the composition is suitable for the introduction into non-human embryonic mammalian cells and their development into a mature immunologically compatible system.

13. The use according to claim 12 wherein said immunologically compatible system comprises a member selected from the group consisting of an organism, an organ, a tissue or cells, or any combination thereof.

## Patentansprüche

1. Nucleinsäurekonstrukt, das eine Vielzahl von Oligonucleotiden umfasst, die komplementär zu verschiedenen Abschnitten einer Ribonucleotidsequenz sind, transkribiert von einem Immunantwort-Gen in einem System, dessen Konstrukt, wenn es in einer Zelle vorliegt, die Funktion des Gens inhibiert oder reguliert, wobei das Immunantwort-Gen codierend ist für: (a) Klasse 1- oder Klasse III-Antigene des Haupthistokompatibilitätskomplexes, oder (b) Zelladhäsionsmoleküle, und wobei die komplementären Oligonucleotide mindestens ein modifiziertes Mitglied ausgewählt aus der Gruppe bestehend aus einem die Assoziationskonstante erhöhenden Modifikator und einem die Assoziation erhöhenden Modifikator oder einer Kombination jeder der Vorgenannten ist.

2. Nucleinsäurekonstrukt nach Anspruch 1, ferner umfassend ein Transkriptionspromotor-Segment und ein Transkriptionsterminations-Segment.

3. Immunologisch kompatibles System, wobei der menschliche Körper ausgeschlossen ist, das ein Nucleinsäurekonstrukt nach Anspruch 1 oder 2 enthält.

4. Immunologisch kompatibles System nach Anspruch 3, umfassend mindestens ein Mitglied ausgewählt aus der Gruppe bestehend aus einem Organismus, einem Organ, einem Gewebe und Zellen oder einer Kombination davon.

5. Immunologisch kompatibles System nach Anspruch 4, wobei der Organismus, das Organ, das Gewebe oder die Zellen aus Säugern sind.

6. Immunologisch kompatibles System nach Anspruch 5, wobei der Organismus, das Organ, das Gewebe oder die Zellen aus Säugern von Affen oder Schweinen sind.

7. Verwendung eines Nucleinsäurekonstrukts nach Anspruch 1 oder 2 zur Herstellung eines Arzneimittels, um ein System immunologisch kompatibel zu machen, wobei das Mittel zur Einbringung in die Zellen des Systems geeignet ist.

8. Verwendung nach Anspruch 7, wobei das immunologisch kompatible System definiert ist nach einem der Ansprüche 4 bis 6.

9. Verwendung nach Anspruch 8, wobei der Einbringungsschritt mit einem Vektor, der das Nucleinsäurekonstrukt enthält, auszuführen ist.

10. Verwendung nach Anspruch 9, wobei der Vektor ein Plasmid oder ein Virus ist.

11. Verwendung nach Anspruch 7, wobei der Einbringungsschritt mit einem Verteilungssystem zur Verteilung des Nucleinsäurekonstrukts in die Zelle auszuführen ist.

12. Verwendung eines Nucleinsäurekonstrukts gemäß der Definition in Anspruch 1 oder 2 zur Herstellung eines Arzneimittels, um ein System immunologisch kompatibel zu machen, wobei das Mittel zur Einbringung in nicht-menschliche embryonale Säugerzellen und deren Entwicklung in ein reifes, immunologisch kompatibles System geeignet ist.

13. Verwendung nach Anspruch 12, wobei das immunologisch kompatible System ein Mitglied umfasst, ausgewählt aus der Gruppe bestehend aus einem Organismus, einem Organ, einem Gewebe oder Zellen oder jeder Kombination davon.

## Revendications

1. Construction d'acide nucléique comprenant une pluralité d'oligonucléotides complémentaires de différentes parties d'une séquence ribonucléotidique transcrite à partir d'un gène de réponse immune dans un système, laquelle construction, lorsqu'elle est présente dans une cellule, inhibe ou régule la fonction dudit gène, ledit gène de réponse immune codant pour : (a) des antigènes de Classe I ou Classe III du complexe majeur d'histocompatibilité, ou (b) des molécules d'adhésion cellulaire, et lesdits oligonucléotides complémentaires comprenant au moins un membre modifié choisi dans le groupe constitué par un modificateur augmentant la constante d'association et un modificateur augmentant l'association, ou une combinaison de précités.

2. Construction d'acide nucléique selon la revendication 1 comprenant en outre un segment de promoteur transcriptionnel et un segment d'arrêt de transcription.

3. Système immunologiquement compatible à l'exception du corps humain contenant une construction d'acide nucléique selon la revendication 1 ou 2.

4. Système immunologiquement compatible selon la revendication 3 comprenant au moins un membre choisi dans le groupe constitué d'un organisme, d'un organe, d'un tissu, et de cellules, ou une combinaison de ceux-ci.

5. Système immunologiquement compatible selon la revendication 4 dans lequel ledit organisme, organe, tissu, ou cellules sont mammifères.

6. Système immunologiquement compatible selon la revendication 5 dans lequel ledit organisme mammifère, organe mammifère, tissu mammifère, ou cellules mammifères sont simiens ou porcins.

7. Utilisation d'une construction d'acide nucléique selon la revendication 1 ou 2 pour la préparation d'une composition pharmaceutique pour rendre un système immunologiquement compatible, ladite composition étant appropriée pour l'introduction dans les cellules dudit système.

8. Utilisation selon la revendication 7, dans laquelle le système immunologiquement compatible est défini selon l'une quelconque des revendications 4 à 6.

9. Utilisation selon la revendication 8, dans laquelle ladite étape d'introduction doit être effectuée avec un vecteur ayant incorporé ladite construction d'acide nucléique.

10. Utilisation selon la revendication 9, dans laquelle ledit vecteur est un plasmide ou un virus.

11. Utilisation selon la revendication 7, dans laquelle ladite étape d'introduction doit être effectuée avec un système de délivrance pour délivrer ladite construction d'acide nucléique à ladite cellule.

12. Utilisation d'une construction d'acide nucléique comme défini dans la revendication 1 ou 2 pour la préparation d'une composition pharmaceutique pour rendre un système immunologiquement compatible, la composition étant appropriée pour l'introduction dans des cellules mammifères embryonnaires non-humaines et leur développement dans un système immunologiquement compatible mature.

13. Utilisation selon la revendication 12, dans laquelle ledit système immunologiquement compatible comprend un membre choisi dans le groupe constitué d'un organisme, d'un organe, d'un tissu, ou de cellules, ou de toute combinaison de ceux-ci.
